# EUROPEAN PATENT APPLICATION

(11) **EP 3 607 969 A1**
(43) Date of publication of application: **12.02.2020**
(21) Application number: 18780466.1
(22) Date of filing: 04.04.2018
(51) Int. Cl.: A61K 39/395, A61K 47/00, A61P 37/00

(54) **INFLIXIMAB COMPOSITION CONTAINING HISTIDINE BUFFER SYSTEM**

(30) Priority: 07.04.2017 CN 201710224525
(71) Applicant: Hisun Bioray Pharmaceutical Co., Ltd., Taizhou, Zhejiang 318000 (CN)
(72) Inventor: FANG, Weijie, Taizhou, Zhejiang 318000 (CN); QIAN, Ci, Taizhou Zhejiang 318000 (CN); WANG, Haibin, Taizhou Zhejiang 318000 (CN); GAO, Dong, Taizhou Zhejiang 318000 (CN)
(74) Representative: Schnappauf, Georg
(86) International application number: PCT/CN2018/081806
(87) International publication number: WO 2018/184540

(57) **Abstract**

Provided is a pharmaceutical composition, which comprises infliximab, a histidine buffer system, a structure protectant and a surfactant, the concentration of histidine in the histidine buffer system being approximately 1-200 mM.

## Description

The present application claims the priority of Chinese Patent Application No. 201710224525.7, filed on April 7, 2017, entitled "Infliximab composition containing histidine buffer system". The content of which is incorporated by reference in its entirety.

### Technical field

The invention belongs to the field of pharmacy, and in particular relates to an infliximab composition comprising a histidine buffer system.

### Background technique

Infliximab monoclonal antibody (infliximab, CAS # 170277-31-3) is a human-mouse chimeric monoclonal antibody that prevents the binding of TNF-α to its cell surface receptor by specifically binding to TNF-α in human, thereby blocking the biological activity of TNF-α, ultimately reduces the inflammatory response and reduces osteoclast activation, achieving the purpose of controlling and alleviating symptoms. Infliximab can be used to treat rheumatoid arthritis, psoriasis, ankylosing spondylitis, ulcerative colitis and Crohn's disease, etc.

Infliximab, however, is not sufficiently stable, and can occur a variety of chemical and physical degradations. In particular, the higher order structure of the infliximab is very fragile, and thus structural changes such as denaturation, aggregation, and precipitation may easily occur. These degraded or unstable products may have a major impact on the safety of biopharmaceuticals. In particular, some protein aggregates may stimulate the body's immune response, which would reduce the efficacy of biopharmaceuticals or even cause death. Therefore, aggregation is considered as a critical quality attribute (CQA) for biopharmaceutical safety, directly affecting the medication safety of infliximab. For antibody drugs, not only high-purity product is required during manufacturing, but also structural stability during transportation, storage, and administration should be maintained.

CN104159614A discloses an improved liquid formulation comprising a suitable amount of a therapeutic protein in a suitable buffer, one or more suitable stabilizers, and other excipients optionally selected from suitable surfactants and tonicity agents. CN103402540A relates to a non-aqueous high concentration suspension comprising a carrier containing a hydrophobic agent and a viscosity reducing agent and an antibody formulated with an excipient.

### Summary of invention

In one aspect, provided is a pharmaceutical composition comprising infliximab, a histidine buffer system, a structure protectant and a surfactant,
wherein in the composition, the concentration of infliximab is from about 5 to about 40 mg/ml, the concentration of histidine in the histidine buffer system is from about 1 to about 200 mM, the concentration of the structure protectant is from about 0 to about 100 mg/ml, and the concentration of the surfactant is from about 0.01 to about 1 mg/ml.

In a preferred embodiment, the concentration of infliximab in the pharmaceutical composition is from about 5 to about 20 mg/ml.

In another preferred embodiment, the concentration of histidine in the pharmaceutical composition is from about 5 to about 75 mM, preferably from about 25 to about 45 mM, e.g., about 25, 30, 35, 40, 45 mM.

In another preferred embodiment, the structure protectant in the pharmaceutical composition is selected from the group consisting of sucrose, trehalose, and combinations thereof, wherein the concentration of the structure protectant is from about 10 to about 75 mg/ml, preferably from about 10 to about 50 mg/ml.

In yet another preferred embodiment, the concentration of the surfactant in the pharmaceutical composition is from about 0.025 to about 0.4 mg/ml, preferably from about 0.025 to about 0.1 mg/ml.

In still another embodiment, the surfactant is selected from the group consisting of polysorbate 20, polysorbate 21, polysorbate 40, polysorbate 60, polysorbate 61, polysorbate 65, polysorbate 80, polysorbate 81, polysorbate 85, poloxamer, triton, sodium dodecyl sulfate, sodium lauryl sulfate, sodium octyl glycoside, lauryl-sulfobetaine, polyethylene glycol, polypropylene glycol, and combinations thereof, preferably polysorbate 80.

In one embodiment, the pH of the pharmaceutical composition is from about 4.0 to about 9.0, preferably from about 4.5 to about 8.0, more preferably from about 5.2 to about 7.2.

In a preferred embodiment, the pharmaceutical composition of the present invention comprises:

| | |
|---|---|
| 1 (1) infliximab | 5 to 20 mg/ml, |
| (2) sucrose | 10 to 50 mg/ml, |
| (3) polysorbate 80 | 0.025 to 0.1 mg/ml, |
| (4) histidine | 25 to 45 mM; |

and the pH is about from 5.2 to 7.2.

In still another aspect, the present invention relates to a method for the prevention or treatment of human autoimmune-associated diseases, comprising administering to a subject in need thereof a pharmaceutical composition of the present invention.

In still another aspect, the invention also relates to the use of a pharmaceutical composition according to the invention for the manufacture of a medicament for the prevention or treatment of human autoimmune-associated diseases.

In still another aspect, the invention also relates to a pharmaceutical composition for use in the prevention or treatment of human autoimmune-associated diseases.

In one embodiment, the human autoimmune-associated diseases include, but are not limited to, rheumatoid arthritis, psoriasis, juvenile idiopathic arthritis, ankylosing spondylitis, Crohn's disease, ulcerative colitis, plaque psoriasis, etc.

### Detailed Description

### General definition and terminology

Unless otherwise stated, the terms and phrases used herein have the meanings listed below. Particular terms or phrases should not be considered as undefined or unclear when they are not specifically defined, and should be interpreted according to what is generally understood by those skilled in the art. When a trade name appears in this document, it is intended to refer to its corresponding product or its active ingredient.

When used with a numerical variable, the term "approximate" or "about" generally refers to the value of the variable and all the values of the variable within the experimental error (for example, within the 95% confidence interval of the mean) or within ± 10% of the specified value, or a wider range.

The expression "comprise" or its synonyms "contain", "include", "have" or the like is open-ended, which does not exclude other unlisted elements, steps or ingredients. The expression "consist of" excludes any unlisted elements, steps or ingredients. The expression "substantially consist of' refers to specified elements, steps or ingredients within a given range, together with optional elements, steps or ingredients which do not substantively affect the basic and novel features of the claimed subject matter. It should be understood that the expression "comprise" encompasses the expressions "substantially consist of' and "consist of'.

The term "optional" or "optionally" as used herein means that the subsequently described event or circumstance may or may not occur, and the description includes the occurrence or non-occurrence of the event or circumstance. For example, the composition optionally includes an antioxidant covering the case with or without an antioxidant.

The percentages, parts, and the like herein are calculated based on weight unless otherwise indicated.

The infliximab used herein is commercially available and has the CAS Number of 170277-31-3, e.g., maybe obtained from Zhejiang Hisun Pharmaceutical Co., Ltd.

The term "histidine buffer" or "histidine buffer system" refers to a buffer system obtained by combining histidine with an acid which is optionally present for pH adjustment, e.g., hydrochloric acid, acetic acid, sulfuric acid and the like. When characterizing a histidine buffer system in a formulation, the sum of the concentrations of histidine, including its free and ionized forms, is typically used. Examples of histidine buffers include, but are not limited to, histidine/hydrochloric acid, histidine/acetic acid, histidine/sulfuric acid, and the like. A preferred histidine buffer of the present invention is a histidine/hydrochloric acid buffer.

The term "carbohydrate" includes monosaccharides, disaccharides, oligosaccharides and polysaccharides. Examples of carbohydrates include, but are not limited to, fructose, glucose, sucrose, trehalose, mannose, lactose, mannose, maltose, sorbose, dextran, dextrin, cyclodextrin, hydroxyethyl starch, or combinations thereof.

The term "amino acid" includes amino acids and/or pharmaceutically acceptable salts thereof. Examples of amino acids include, but are not limited to, alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine or combinations thereof.

"Pharmaceutically acceptable salt" refers to a salt formed with a pharmaceutically acceptable non-toxic base or acid, including inorganic or organic bases and inorganic or organic acids. For example, a salt formed with the following inorganic acids: for example, hydrochloric acid, hydrobromic acid, hydroiodic acid, sulfuric acid, phosphoric acid or nitric acid; or a salt formed with the following organic acids: for example, formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, nicotinic acid, pamoic acid, picric acid, lauryl sulfate, ethanesulfonic acid, benzenesulfonic acid, p-toluenesulfonic acid, methanesulfonic acid, citric acid, tartaric acid, stearic acid, lactic acid, oxalic acid, malonic acid, succinic acid, malic acid, alginic acid, maleic acid, fumaric acid, D-gluconic acid, mandelic acid or ascorbic acid.

The term "lyophilization" refers to a drying technique, in which a solution formulation is frozen to a solid state at a lower temperature, and then the water therein is sublimed directly to a gaseous state under vacuum without passing through a liquid state, and finally the material is dehydrated. Lyophilization includes, but is not limited to, freezing, primary drying, and secondary drying.

### Pharmaceutical composition and administration

The pharmaceutical composition of the present invention comprises a histidine buffer system. The stability of the infliximab pharmaceutical composition comprising a histidine buffer system is significantly improved compared to the phosphate buffer system used in the prior art.

Specifically, provided is a pharmaceutical composition comprising infliximab, a histidine buffer system, a structure protectant, and a surfactant.

In one embodiment, the concentration of infliximab in the pharmaceutical composition is from about 5 to about 40 mg/ml, preferably from about 5 to about 20 mg/ml.

In the formulation of the present invention, examples of the histidine buffer system include, but are not limited to, histidine/hydrochloric acid, histidine/acetic acid, histidine/sulfuric acid, and the like, preferably histidine/hydrochloric acid buffer. In one embodiment, the concentration of histidine in the histidine buffer system of the pharmaceutical composition is from about 1 to about 200 mM, preferably from about 5 to about 75 mM, more preferably from about 25 to about 45 mM, e.g., about 25, 30, 35, 40, 45 mM.

The pharmaceutical composition of the present invention may comprise a structure protectant. The structure protectant can be used to stabilize the structure of the active ingredient in the pharmaceutical composition, providing protection at room temperature and lyophilization conditions. Carbohydrates and amino acids are commonly used stabilizers for protein drugs. It also acts as a cryoprotectant and a desiccant to protect the protein from denaturation that may occur during lyophilization. In one embodiment, the structure protectant is carbohydrate, amino acid, or combinations thereof. In a preferred embodiment, the structure protectant is selected from the group consisting of sucrose, trehalose, and combinations thereof, more preferably sucrose. The concentration of the structure protectant is from about 0 to about 100 mg/ml, preferably from about 10 to about 75 mg/ml, more preferably from about 10 to about 50 mg/ml, e.g., about 25 mg/ml.

The pharmaceutical formulation of the present invention may further comprise a surfactant. The surfactant is a substance that can change (usually reduce) the surface tension of a liquid or the interfacial tension between two phases. The surfactant is amphiphilic and contains a hydrophilic group and a lipophilic group. Examples of the surfactant include, but are not limited to, polysorbates, poloxamer, tritons, sodium dodecyl sulfate, sodium lauryl sulfate, sodium octyl glycoside, lauryl-sulfobetaine, polyethylene glycol, polypropylene glycol or mixtures thereof. Polysorbate surfactants which can be used are polysorbate 20, polysorbate 21, polysorbate 40, polysorbate 60, polysorbate 61, polysorbate 65, polysorbate 80, polysorbate 81, polysorbate 85 or a mixture thereof, preferably polysorbate 80. In the pharmaceutical composition of the present invention, the concentration of the surfactant may range from about 0.01 to about 1 mg/ml, preferably from about 0.025 to about 0.4 mg/ml, and most preferably from about 0.025 to about 0.1 mg/ml.

The pharmaceutical formulation of the present invention may optionally contain an antioxidant which inhibits the oxidation of the pharmaceutical formulation, improves the stability of the drug and contributes to prolonging the storage period. Examples of antioxidants include, but are not limited to, ascorbic acid, tryptophan, methionine, glutathione, sodium thiosulfate, catalase, or mixtures thereof.

The pharmaceutical composition of the present invention, as required, may optionally comprise a chelating agent which may combine with the metal ion which readily catalyzes the oxidation of the protein, and enhances the stability of the pharmaceutical composition thereby. The chelating agents include but are not limited to aminopolycarboxylic acid, hydroxyaminocarboxylic acid, N-substituted glycine, citric acid, nicotinamide, deferoxamine and deoxycholate and mixtures thereof, e.g., ethylenediaminetetraacetic acid (EDTA), diethylenetriaminepentaacetic acid (DTPA), nitrilotriacetic acid (NTA), and salts thereof. The chelating agent used may be in the form of free acid, free base or the salts thereof, or it may be in the form of anhydrate, hydrate or other solvates of the compound or the corresponding salt.

The pharmaceutical formulation of the present invention may also optionally contain a preservative, which inhibits the growth and reproduction of microorganisms, including, but not limited to, m-cresol, phenol, benzyl alcohol, benzalkonium chloride, phenoxyethanol, p-hydroxybenzoate, and mixtures thereof.

The selection of pH is critical to the stability of the composition and the pH should be chosen such that the corresponding active ingredient remains stable. In addition, the pH should be chosen such that other ingredients in the pharmaceutical composition retain their physical and chemical properties, e.g., without degradation. As a preferred embodiment, a suitable pH is from about 4.0 to about 9.0, preferably from about 4.5 to about 8.0, more preferably from about 5.2 to about 7.2, e.g., about 5.7, 6.7.

The pharmaceutical composition of the present invention may not require an additional addition of a tonicity agent such as sodium chloride, potassium chloride or the like.

In the present invention, a pharmaceutical composition is formulated based on an aqueous solution or water, e.g., water, for injection.

In a specific embodiment, provided is a pharmaceutical composition comprising:

| | |
|---|---|
| (1) infliximab | 5 to 20 mg/ml, |
| (2) sucrose | 10 to 50 mg/ml, |
| (3) polysorbate 80 | 0.025 to 0.1 mg/ml, |
| (4) histidine | 25 to 45 mM; |

and the pH is about from 5.2 to 7.2.

The pharmaceutical composition of the present invention may be an injection formulation, a lyophilized formulation, or a formulation form prepared by a lyophilized powder and water for injection through a dual-chamber cartridge, which may be administered by subcutaneous injection (s.c.), intravenous injection (i.v.), intramuscular injection (i.m.) or other modes of parenteral administration.

### Beneficial effects

The invention adopts histidine as a buffer system for infliximab, which greatly improves the stability of the pharmaceutical composition and its lyophilized formulation, especially under high temperature and light exposure conditions, and the pharmaceutical composition can be stable for long-term storage in the lyophilization state.

### Examples

The invention is further illustrated by the following examples. These examples are intended to illustrate the invention and are not to be construed as limiting the invention. In this context, the proportions including percentages are calculated based on weight unless otherwise indicated or apparently unsuitable. "/" means without containing or not detected.

### Preparation

The solution of the infliximab pharmaceutical composition was prepared by using water for injection, and the product was subjected to lyophilization, and the lyophilization process is shown in Table 1.

**Table 1. Infliximab lyophilization process**

| | Shelf temperature (°C) | Conversion time (min) | Maintenance time (min) | Chamber pressure |
|---|---|---|---|---|
| Prefreeze | 5 | N/A | 60 | Atmospheric pressure |
| | -40 | 100 | 90 | Atmospheric pressure |
| | -5 | 80 | 120 | Atmospheric pressure |
| | -40 | 80 | 90 | Atmospheric pressure |
| Primary drying (sublimation) | -20 | 125 | 1440 | 0.075 mbar |
| Secondary drying (desorption) | 25 | 250 | 600 | 0.075 mbar |

High temperature and light exposure experiments were performed on the formulations before and after lyophilization, respectively.

High-temperature experiment: the sample was placed in an incubator at 40 °C ± 2 °C, and was sampled and measured at the first week and the second week, or at the second week and the fourth week, respectively;
Light exposure experiment: the sample was placed in an illumination box (5 °C ± 3 °C, 4500 Lx ± 500 Lx) and was sampled 10 days later.

### Reagents and testing instruments

### Reagents:

Infliximab, provided by Zhejiang Hisun Pharmaceutical Co., Ltd.
Histidine, purchased from JT Baker
Sucrose, purchased from Merck KGaA
Trehalose, purchased from HAYASHIBARA Co., Ltd., Japan
Arginine hydrochloride, purchased from JT Baker
Polysorbate 80, purchased from JT Baker

### Instruments:

Agilent 1200 liquid chromatography, purchased from Agilent, USA.
SEC-HPLC chromatographic column: TSK-GEL G3000 SWXL from TOSOH, Japan, 7.8×300 mm; mobile phase: 0.02 mol/L sodium dihydrogen phosphate, 0.2 mol/L sodium chloride buffer, 10% acetonitrile, pH 7.0; flow rate: 0.5 ml/min; column temperature: 30 °C; running time: 30 min; wavelength: 280 nm.
Malvern Zetasizer Nano-ZS ZEN3600 is a Dynamic Light Scattering Instrument, purchased from Malvern.

### Example 1

This example compares the effects of a commercially available formulation (Formulation 1), and the formulation using a histidine buffer instead of a phosphate buffer (Formulation 2) on the stability of the infliximab pharmaceutical composition (Table 2).

**Table 2. Composition of the pharmaceutical compositions**

| Serial number | Infliximab (mg/ml) | Sodium phosphate (mM) | Histidine (mM) | Polysorbate 80 (mg/ml) | Sucrose (mg/ml) | pH |
|---|---|---|---|---|---|---|
| Formulation 1 | 20 | 20 | / | 0.1 | 100 | 7.2 |
| Formulation 2 | 20 | / | 20 | 0.1 | 100 | 7.2 |

| | | | | | | |
|---|---|---|---|---|---|---|
| Formulation 1 and Formulation 2 in Table 1 were lyophilized. | | | | | | |

The appearance and visible foreign matter were detected for Formulations 1-2, and the content of aggregation was detected by SEC-HPLC. The results are shown in Table 3.

**Table 3. Appearance/visible foreign matter and the content of aggregation in Formulation 1 and Formulation 2 by high temperature and light exposure**

| Serial number | | Appearance/visible foreign matter | Content of aggregation (%) |
|---|---|---|---|
| Formulation 1 (Injection formulation) | 1 week | Colorless and clear liquid, no foreign matter | / |
| | 2 weeks | Colorless and clear liquid, no foreign matter | 1.15±0.15 |
| | light exposure | Colorless and clear liquid, no foreign matter | 3.78±0.18 |
| Formulation 2 (Injection formulation) | 1 week | Colorless and clear liquid, no foreign matter | / |
| | 2 weeks | Colorless and clear liquid, no foreign matter | 0.82±0.05 |
| | light exposure | Colorless and clear liquid, no foreign matter | 1.01±0.02 |
| Formulation 1 (lyophilized formulation) | 1 week | White block solid, and colorless and clear liquid with no foreign matter after being dissolved | 1.06±0.04 |
| | 2 weeks | White block solid, and colorless and clear liquid with no foreign matter after being dissolved | 0.85±0.02 |
| | light exposure | White block solid, and colorless and clear liquid with no foreign matter after being dissolved | 0.96±0.03 |
| Formulation 2 (lyophilized formulation) | 1 week | White block solid, and colorless and clear liquid with no foreign matter after being dissolved | 0.46±0.00 |
| | 2 weeks | White block solid, and colorless and clear liquid with no foreign matter after being dissolved | 0.46±0.00 |
| | light exposure | White block solid, and colorless and clear liquid with no foreign matter after being dissolved | 0.67±0.03 |

| | | | |
|---|---|---|---|
| * The above and below experimental data is shown as mean ± standard deviation, repeated 3 times. | | | |

The content of aggregation is commonly used to indicate the stability of the formulation. The higher the aggregation content, the worse the formulation stability. The results in Table 3 show that with the use of histidine (Formulation 2) instead of phosphate (Formulation 1) as a buffer solution, the stability of the injection formulation is significantly improved, and the content of aggregation is lower. The stability of the formulations under lyophilization condition is better than that of the injection formulations, and the stability of lyophilized Formulation 2 is even better.

### Example 2

This example compares the effect of buffer species on the stability of the infliximab pharmaceutical composition. The compositions of Formulations 3-4 of the pharmaceutical composition are shown in Table 4.

**Table 4. Composition of the pharmaceutical compositions**

| Serial number | Infliximab (mg/ml) | Sodium phosphate (mM) | Histidine (mM) | Polysorbate 80 (mg/ml) | Sucrose (mg/ml) | pH |
|---|---|---|---|---|---|---|
| Formulation 3 | 5 | 5 | / | 0.1 | 25 | 7.2 |
| Formulation 4 | 5 | / | 5 | 0.1 | 25 | 7.2 |

### Comparison of the particle size of infliximab in aqueous solution

The particle size of the monoclonal antibodies in aqueous solution of the two formulations was investigated using dynamic light scattering (Malvern Zetasizer Nano-ZS ZEN3600) (Table 5). The average particle size of the phosphate-containing Formulation 3 is larger than that of the histidine-containing Formulation 4, indicating that the interaction of the monoclonal antibodies in the histidine solution is weaker, and it is less likely to collide to form aggregation. The stability of the monoclonal antibody in the histidine solution is better.

**Table 5. Particle size of Formulations 3-4 in aqueous solution**

| Serial number | Particle size (nm) |
|---|---|
| Formulation 3 | 20.14 |
| Formulation 4 | 18.75 |

Formulation 3 and Formulation 4 were lyophilized according to the lyophilization process in Table 1. The stabilities of the lyophilized formulation at high temperature (40 °C ± 2 °C, 2 weeks, 4 weeks) and light exposure (5 °C ± 3 °C, 4500 Lx ± 500 Lx, 10 days) conditions were compared (Table 6).

**Table 6. Appearance/visible foreign matter and the content of aggregation in lyophilized Formulation 3 and Formulation 4 by high temperature and light exposure**

| Serial number | | Appearance/visible foreign matter | Content of aggregation (%) |
|---|---|---|---|
| Formulation 3 | 2 weeks | White block solid, and colorless and clear liquid with no foreign matter after dissolution | 1.71±0.06 |
| | 4 weeks | White block solid, and colorless and clear liquid with no foreign matter after dissolution | 2.03±0.03 |
| | light exposure | White block solid, and colorless and clear liquid with no foreign matter after dissolution | 1.13±0.06 |
| Formulation 4 | 2 weeks | White block solid, and colorless and clear liquid with no foreign matter after dissolution | 1.30±0.02 |
| | 4 weeks | White block solid, and colorless and clear liquid with no foreign matter after dissolution | 1.52±0.04 |
| | light exposure | White block solid, and colorless and clear liquid with no foreign matter after dissolution | 0.98±0.03 |

After high temperature or light exposure process, lyophilized Formulation 4 formulated in a histidine buffer, compared to lyophilized Formulation 3 formulated in a phosphate buffer, has a lower content of aggregation, i.e. higher stability.

### Example 3

In this example, each of the infliximab pharmaceutical compositions (Formulations 5-8) was prepared according to Table 7, wherein the pH was adjusted with hydrochloric acid. Formulations 5-8 were lyophilized using the lyophilization process in Table 1. After lyophilization, high temperature (40 ± 2 °C) and light exposure (5 °C ± 3 °C, 4500 Lx ± 500 Lx, 10 days) experiments were performed.

**Table 7. Composition of the pharmaceutical compositions**

| Serial number | Infliximab (mg/ml) | Histidine (mM) | Sucrose (mg/ml) | Polysorbate 80 (mg/ml) | pH |
|---|---|---|---|---|---|
| Formulation 5 | 5 | 5 | 25 | 0.1 | 7.2 |
| Formulation 6 | 5 | 5 | 25 | 0.1 | 6.7 |
| Formulation 7 | 5 | 5 | 25 | 0.1 | 5.7 |
| Formulation 8 | 5 | 5 | 25 | 0.1 | 4.7 |

The lyophilized formulations are white block solid in appearance after being subjected to high temperature or light exposure process, and are colorless clear liquid after dissolution, with no visible foreign matter. The content of the aggregation in Formulations 5-8 was determined by SEC-HPLC after the experiments, and the results are shown in Table 8.

**Table 8. The content of aggregation (%) of Formulations 5-8 after high temperature and light exposure process**

| Serial number | Day 0 | 40°C for 2 week | 40 °C for 4 weeks | light exposure for 10 days |
|---|---|---|---|---|
| Formulation 5 | 0.91±0.02 | 1.30±0.02 | 1.38±0.04 | 0.98±0.03 |
| Formulation 6 | 0.91±0.04 | 1.23±0.02 | 1.28±0.03 | 0.98±0.03 |
| Formulation 7 | 0.82±0.02 | 1.12±0.03 | 1.17±0.02 | 0.90±0.02 |
| Formulation 8 | 0.79±0.01 | 1.01±0.06 | 1.10±0.04 | 0.82±0.02 |

It can be seen from Table 8 that after the high temperature process of 40 °C, the higher the pH, the more obvious the increase of aggregation content. However, the formulation has a high acidity at a pH of 4.7, which is likely to cause pain during injection. Under light exposure conditions, the increase in aggregation content of formulations at different pH is not significant.

### Example 4

This example compares the effect of structure protectants on the stability of the infliximab pharmaceutical composition. Each of the infliximab pharmaceutical compositions (Formulations 9-11) was formulated according to Table 9, and lyophilization was performed using the lyophilization process in Table 1. High temperature (40 ± 2 °C) and light exposure (4500 ± 500 Lx) experiments were performed after lyophilization.

**Table 9. Composition of the pharmaceutical compositions**

| Serial number | Infliximab (mg/ml) | Histidine (mM) | Structure protectant | Polysorbate 80 (mg/ml) | pH |
|---|---|---|---|---|---|
| Formulation 9 | 5 | 5 | 25.0 mg/ml sucrose | 0.1 | 7.2 |
| Formulation 10 | 5 | 5 | 27.6 mg/ml trehalose | 0.1 | 7.2 |
| Formulation 11 | 5 | 5 | 7.7 mg/ml arginine hydrochloride | 0.1 | 7.2 |

The lyophilized formulations are white block solid in appearance after being subjected to high temperature or light exposure process, and are a colorless clear liquid after dissolution, with no visible foreign matter. The aggregation content of Formulations 9-11 was determined by SEC-HPLC after the experiments, and the results are shown in Table 10.

**Table 10. The content of aggregation (%) of Formulations 9-11 after high temperature and light exposure**

| Serial number | Day 0 | 40 °C for 2 weeks | 40 °C for 4 weeks | Light exposure for 10 days |
|---|---|---|---|---|
| Formulation 9 | 0.91±0.02 | 1.30±0.02 | 1.38±0.04 | 0.98±0.03 |
| Formulation 10 | 1.05±0.00 | 1.11±0.03 | 1.17±0.02 | 1.13±0.03 |
| Formulation 11 | 1.19±0.01 | 1.99±0.12 | 2.28±0.11 | 1.38±0.04 |

It can be seen from Table 10 that after high temperature or light exposure experiment, sucrose and trehalose have the same stabilizing effect on the infliximab pharmaceutical composition (the increase of the content of aggregation in Formulations 9, 10 is not obvious), and the stabilizing effect of both sucrose and trehalose are better than arginine hydrochloride.

### Example 5

This example compares the effect of the surfactant concentration on the stability of the infliximab pharmaceutical composition. Each of the infliximab pharmaceutical compositions (Formulations 12-14) was formulated according to Table 11, and Formulations 12-14 was lyophilized with the lyophilization process of Table 1. High temperature (40 ± 2 °C) and light exposure (4500 ± 500 Lx) experiments were performed after lyophilization.

**Table 11. Composition of the pharmaceutical compositions**

| Serial number | Infliximab (mg/ml) | Histidine (mM) | Sucrose (mg/ml) | Polysorbate 80 (mg/ml) | pH |
|---|---|---|---|---|---|
| Formulation 12 | 5 | 5 | 25 | 0.1 | 7.2 |
| Formulation 13 | 5 | 5 | 25 | 0.025 | 7.2 |
| Formulation 14 | 5 | 5 | 25 | 0.4 | 7.2 |

The lyophilized formulations are white block solid in appearance after being subjected to high temperature or light exposure process, and are a colorless clear liquid after dissolution, with no visible foreign matter. The content of the aggregation of Formulations 12-14 was determined by SEC-HPLC after the experiments, and the results are shown in Table 12.

**Table 12. The content of aggregation (%) of Formulations 12-14 after high temperature and light exposure process**

| Serial number | Day 0 | 40 °C for 2 weeks | 40 °C for 4 weeks | Light exposure for 10 days |
|---|---|---|---|---|
| Formulation 12 | 0.91±0.02 | 1.30±0.02 | 1.38±0.04 | 0.98±0.03 |
| Formulation 13 | 1.11±0.08 | 1.02±0.07 | 1.10±0.04 | 1.08±0.02 |
| Formulation 14 | 1.03±0.03 | 1.74±0.15 | 1.81±0.04 | 1.12±0.02 |

As can be seen from Table 12, the increase in aggregation content is not significant for the concentration of polysorbate 80 in the range of 0.025-0.4 mg/ml, especially in the range of 0.025-0.1 mg/ml, and the stability of the infliximab pharmaceutical compositions is within an acceptable range.

### Example 6

This example compares the effect of different concentrations of the histidine buffer system on the stability of the infliximab pharmaceutical composition. The infliximab pharmaceutical compositions (Formulations 15-21) were formulated according to Table 13, and the high temperature (40 ± 2 °C) and light exposure (4500 ± 500 Lx) experiments were performed.

**Table 13. Composition of the pharmaceutical compositions**

| Serial number | Infliximab (mg/ml) | Histidine (mM) | Polysorbate 80 (mg/ml) | pH |
|---|---|---|---|---|
| Formulation 15 | 5 | 5 | 0.1 | 7.2 |
| Formulation 16 | 5 | 25 | 0.1 | 7.2 |
| Formulation 17 | 5 | 45 | 0.1 | 7.2 |
| Formulation 18 | 5 | 75 | 0.1 | 7.2 |
| Formulation 19 | 5 | 100 | 0.1 | 7.2 |
| Formulation 20 | 5 | 150 | 0.1 | 7.2 |
| Formulation 21 | 5 | 178 | 0.1 | 7.2 |

After the experiments, a test for appearance/visible foreign matter was performed, and the aggregation content was detected by SEC-HPLC. The results are shown in Table 14.

As shown in Table 14, with the increase of the concentration of histidine, the aggregation content gradually decreases (Formulations 15-19) and then gradually increases (Formulation 20-21) under high temperature conditions. The stability of the formulation under light exposure conditions is more sensitive to the change of histidine concentration. As the concentration of histidine increases, the aggregation content of Formulations 15-17 gradually decreases, and the aggregation content of Formulations 18-20 gradually increases. In addition, Formulations 19-21 have a darker color after 2 weeks of high temperature or light exposure process, and some samples have foreign matters visible with the naked eye. The stability results of Formulations 16-18, especially Formulation 17, are particularly excellent among the tested formulations.

**Table 14. The content of aggregation (%) of Formulations 15-21 after high temperature and light exposure**

| Serial number | | Appearance / visible foreign body | Content of aggregation (%) |
|---|---|---|---|
| Formulation 15 | 1 week | Colorless and clear liquid, no foreign matter | 0.15±0.01 |
| | 2 weeks | Colorless and clear liquid, no foreign matter | 0.32±0.01 |
| | Light exposure | Colorless and clear liquid, no foreign matter | 0.77±0.06 |
| Formulation 16 | 1 week | Colorless and clear liquid, no foreign matter | 0.13±0.00 |
| | 2 weeks | Colorless and clear liquid, no foreign matter | 0.27±0.01 |
| | Light exposure | Colorless and clear liquid, no foreign matter | 0.57±0.03 |
| Formulation 17 | 1 week | Colorless and clear liquid, no foreign matter | 0.12±0.01 |
| | 2 weeks | Colorless and clear liquid, no foreign matter | 0.27±0.01 |
| | Light exposure | Colorless and clear liquid, no foreign matter | 0.52±0.07 |
| Formulation 18 | 1 week | Colorless and clear liquid, no foreign matter | 0.12±0.0 1 |
| | 2 weeks | Colorless and clear liquid, no foreign matter | 0.24±0.01 |
| | Light exposure | Colorless and clear liquid, no foreign matter | 0.58±0.05 |
| Formulation 19 | 1 week | Colorless and clear liquid, no foreign matter | 0.11±0.01 |
| | 2 weeks | Slightly yellow color, no foreign matter | 0.24±0.00 |
| | Light exposure | The color begins to turn yellow with no foreign matter | 0.79±0.02 |
| Formulation 20 | 1 week | Slightly yellow color, no foreign matter | 0.12±0.00 |
| | 2 weeks | Obviously yellow color, partially with white strips of granular foreign matters. | 0.27±0.01 |
| | Light exposure | The color turns yellow with no foreign matter | 1.04±0.06 |
| Formulation 21 | 1 week | Slightly yellow color, no foreign matter | 0.13±0.00 |
| | 2 weeks | Obviously yellow color, no foreign matter | 0.30±0.01 |
| | Light exposure | Yellower color, no foreign matter | 0.98±0.08 |

Unless otherwise stated, all numbers expressing quantities of ingredients, cell culture, processing conditions, and the like, used in the specification (including the claims) are to be understood as being modified by the term "about" under all conditions. Accordingly, unless indicated to the contrary, the numerical parameters are approximate and may vary depending on the desired characteristics sought to be obtained by the present invention. Unless otherwise stated, the term "at least" preceding a list of elements shall be understood to mean each element in the series. Those skilled in the art will recognize, or be able to devise many equivalents of the specific embodiments of the invention described herein without departing from routine experimentation. The appended claims are intended to cover such equivalents.

Those skilled in the art will appreciate that many modifications and variations of the present invention will be made without departing from the spirit and scope thereof. The specific embodiments described herein are offered by way of examples only and are not intended to be limiting. The true scope and spirit of the invention are shown in the appended claims, and the description and examples are only exemplary.

## Claims

1. A pharmaceutical composition comprising infliximab, a histidine buffer system, a structure protectant, and a surfactant, wherein, in the composition,
the concentration of infliximab is from 5 to 40 mg/ml,
the concentration of histidine in the histidine buffer system is from 1 to 200 mM,
the concentration of the structure protectant is from 0 to 100 mg/ml, and
the concentration of the surfactant is from 0.01 to 1 mg/ml.

2. The pharmaceutical composition according to claim 1, **characterized in that**,
the concentration of infliximab is from 5 to 20 mg/ml.

3. The pharmaceutical composition according to claim 1 or 2, **characterized in that**,
the concentration of histidine is from 5 to 75 mM, preferably from 25 to 45 mM, more preferably 45 mM.

4. The pharmaceutical composition according to any one of claims 1 to 3, **characterized in that**,
the structure protectant is selected from the group consisting of sucrose, trehalose and combinations thereof, wherein the concentration of the structure protectant is from 10 to 75 mg/ml, preferably from 10 to 50 mg/ml.

5. The pharmaceutical composition according to any one of claims 1 to 4, **characterized in that**,
the concentration of the surfactant is from 0.025 to 0.4 mg/ml, preferably from 0.025 to 0.1 mg/ml.

6. The pharmaceutical composition of claim 5, **characterized in that**,
the surfactant is selected from the group consisting of polysorbate 20, polysorbate 21, polysorbate 40, polysorbate 60, polysorbate 61, polysorbate 65, polysorbate 80, polysorbate 81, polysorbate 85, poloxamer, triton, sodium dodecyl sulfate, sodium lauryl sulfate, sodium octyl glycoside, lauryl-sulfobetaine, polyethylene glycol, polypropylene glycol and combinations thereof, preferably polysorbate 80.

7. The pharmaceutical composition according to any one of claims 1 to 6, **characterized in that**,
the pharmaceutical composition has a pH of from 4.0 to 9.0, preferably from 4.5 to 8.0, more preferably from 5.2 to 7.2.

8. Use of a pharmaceutical composition according to any one of claims 1 to 7 for the manufacture of a medicament for the prevention or treatment of human autoimmune-associated diseases.

9. The use according to claim 8, **characterized in that**,
the diseases comprise rheumatoid arthritis, psoriasis, juvenile idiopathic arthritis, ankylosing spondylitis, Crohn's disease, ulcerative colitis, plaque psoriasis.
